(19) Europäisches Patentamt
European Patent Office
Office européen des brevets

(11) Veröffentlichungsnummer: **0 193 787**
**A2**

(12) **EUROPÄISCHE PATENTANMELDUNG**

(21) Anmeldenummer: **86102094.9**

(22) Anmeldetag: **18.02.86**

(51) Int. Cl.[4]: **C 07 C 119/048**, C 08 G 18/79, C 08 G 18/72

(30) Priorität: **26.02.85 US 705743**

(43) Veröffentlichungstag der Anmeldung: **10.09.86 Patentblatt 86/37**

(84) Benannte Vertragsstaaten: **BE DE FR GB IT**

(71) Anmelder: **BASF Corporation, 9 Campus Drive, Parsippany, NJ 07054 (US)**

(72) Erfinder: **Lamboy, Osvaldo, 57 Toby Drive, Succasunna New Jersey 07876 (US)**
Erfinder: **Lightsey, John William, 13508 Rampart Ct., Baton Rouge Louisiana 70810 (US)**
Erfinder: **Kan, Peter Tai-Yuen, 47200 Ann Arbor Road, Plymouth Michigan 48170 (US)**

(74) Vertreter: **Welzel, Dr. Gunther et al, c/o BASF Aktiengesellschaft Carl-Bosch-Strasse 38, D-6700 Ludwigshafen (DE)**

(54) **Stabile 4,4'-Diphenylmethandiisocyanate.**

(57) Stable 4,4'-diphenylmethane diisocyanates are composed of 4,4'-diphenylmethane diisocyanates containing from about 2 to about 10 weight percent carbodiimide-uretonimine structure. The diisocyanates are useful in preparing polyurethane foams.

EP 0 193 787 A2

STABLE 4,4'-DIPHENYLMETHANE DIISOCYANATES

The present invention concerns low temperature stable organic polyisocyanates. More particularly, the present invention relates to stable diphenylmethane diisocyanates compositions and to polyurethane foams prepared therefrom.

U.S. Patent 3,449,256 teaches a process for the preparation of polyisocyanates stable at a temperature of about 15°C. The present invention relates to compositions of polyisocyanates which are stable at temperature of 0°C for at least two weeks.

Low temperature stable organic polyisocyanates are prepared by partially reacting 4,4'-diphenylmethane diisocyanate in the presence of an effective amount of a carbodiimidization catalyst. The reaction proceeds to the extent that the polyisocyanate has a uretonimine content from about 2 to 10 weight percent. The polyisocyanate compositions may also be prepared by blending various diisocyanates to give the desired compositions.

In accordance with the present invention, carbodiimide-uretonimine modified organic diphenylmethane diisocyanates useful for the preparation of polyurethane foam products and the like are prepared by heating 4,4'-diphenylmethane diisocyanate in the presence of a catalytically effective amount of a carbodiimidization catalyst. The reaction proceeds to the extent that the diphenylmethane diisocyanate has a carbodiimide-uretonimine content from about 2 to about 10 weight percent. The catalyst is generally employed at a concentration ranging from 0.0004 part to 5.0 parts per 100 parts of organic polyisocyanates. Preferably, the catalyst is employed at a concentration ranging from 0.0004 to 1 part per 100 parts of the polyisocyanate. The temperatures employed are those over 30°C. Generally, the range is 50 to 250°C, preferably from about 50 to 120°C. and 200 to 230°C. The time required for carrying out this reaction is dependent upon the quantity of catalyst employed. However, the time can vary from about 0.5 hour to 6 hours. When the catalyst is employed in the preferred amount, the reaction time ranges from 0.5 hour to 4 hours. After the required reaction time period has elapsed the resulting product is cooled to temperatures less than 30°C. Optionally, the reaction product may be treated at the reaction temperature or lower with catalyst deactivators which include salts such as magnesium chloride dihydrate, acid chlorides such as benzoyl chlorides and acetyl chlorides, acids such as hydrochloric acid, oxalic acid, phosphoric acid, benzenesulfonic acid, toluenesulfonic acid, methanesulfonic acid or trifluoromethanesulfonic acid, sulfonyl chlorides such as benzenesulfonyl chloride, toluenesulfonyl chloride and the like. Other deactivators which may be employed are such agents as dimethylsulfate, alkyl o,p-toluenesulfonates, methyl chloride and similar compounds as disclosed in U. S. Patent 3,769,318.

The preferred composition is also prepared by blending a carbodiimide-uretonimine containing 4,4'-diphenylmethane diisocyanate with pure 4,4'-diphenylmethane diisocyanate wherein the blended composition contains from about 2 to about 10 weight percent carboiimide-uretonimine structure.

In the presence of excess polyisocyanate, the carbodiimide and uretonimine structure exists in equilibrium as shown below.

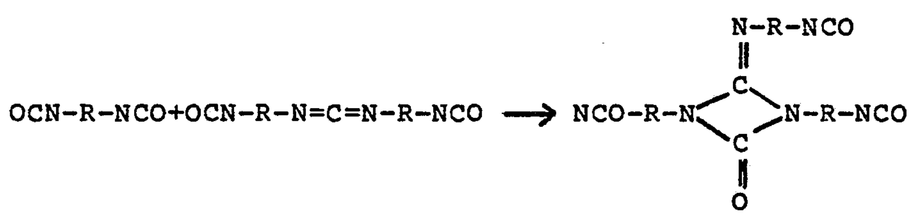

wherein R is diphenylmethane

The organic diisocyanates of the present invention are useful in the preparation of rigid, semi-flexible, or flexible polyurethane foams. The polyurethane foams in accordance herewith are prepared by reacting the organic diisocyanate and an active hydrogen-containing compound in the presence of urethane catalysts as are well known to those skilled in the art.

The polyisocyanate compositions of the instant invention may be prepared by employing well-known carbodiimide-promoting compounds as catalysts. The carbodiimide catalysts employed in accordance with the invention can be any of those known in the art as being useful in the conversion of an isocyanate to the corresponding carbodiimide. Illustrative of such catalysts are:

a) phospholene 1-oxides and 1-sulfides having the formulae:

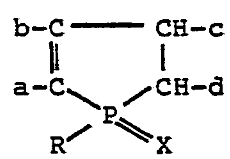

and

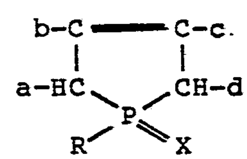

wherein a, b, c and d are each selected from the group consisting of hydrogen and hydrocarbyl from 1 to 12 carbon atoms inclusive, R is selected from the group consisting of lower alkyl and aryl and X is selected from the group consisting of oxygen and sulfur. The above phospholene

compounds and methods for their preparation are described in U. S. Patents 2,663,737; 2,663,738; and 2,853,473. The 3-phospholenes can be isomerized readily to the corresponding 2-phospholenes by thermal-treatment or by refluxing with an aqueous based as disclosed by Quinn et al, Journal American Chemical Society, 33, 1024, 1968. Representative compounds within the above class are 1-phenyl-2-phospholene-1-oxide; 3-methyl-1-phenyl-3-phospholene-1-oxide; 3-methyl-1-phenyl-2-phospholene-1-oxide; 1-phenyl-2-phospholene-1-sulfide; 1-ethyl-2-phospholene-1-oxide; 1-ethyl-3-methyl-2-phospholene-1-oxide; 1-ethyl-3-methyl-2-phospholene-1-sulfide; and the isomeric phospholanes corresponding to the above-named compounds. Also, polymer bound phospholene oxide may be employed specifically those having recurring units, for example,

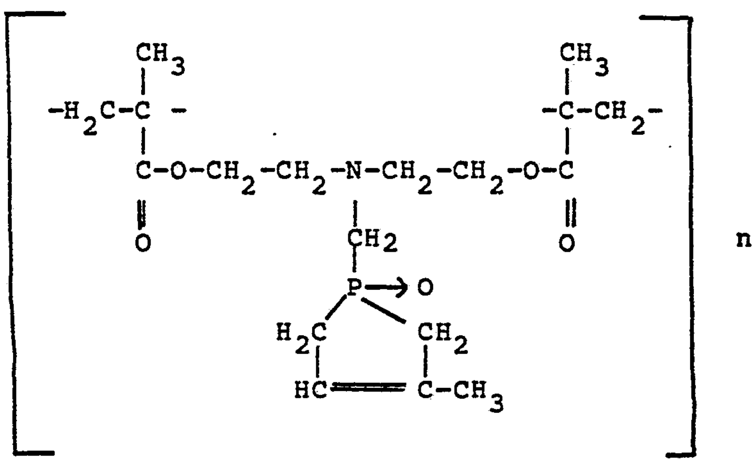

as disclosed in U. S. Patent 4,105,643, and those of the following structure as disclosed in U. S. Patent 4,105,642.

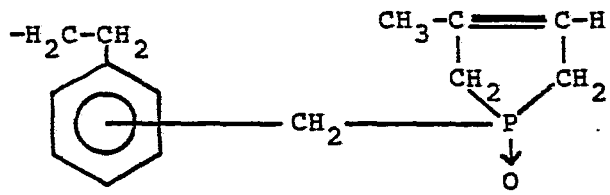

(b) diaza- and oxaza-phospholanes and -phosphorinanes

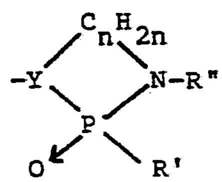

wherein $C_nH_{2n}$ represents alkylene from 1 to 12 carbon atoms, inclusive, at least one and not more than three adjacent carbon atoms and said alkylene radical forming a chain, one end of which is attached to Y, the other end of which is attached to N, thereby completing the heterocyclic ring; R' is selected from the group consisting of hydrocarbyl containing 1 to 12 carbon atoms, inclusive; and halo, nitro, alkoxy, alkyl, mercapto, and cyano-substituted hydrocarbyl from 1 to 12 carbon atoms, inclusive; R" is hydrocarbyl containing from 1 to 12 carbon atoms, inclusive, and Y is selected from the group consisting of -O- and -NR"- wherein R" has the significance as defined above. The above compounds and methods for their preparation are described in

U. S. Patent 3,522,303. Representative examples of such compounds are: 2-ethyl-1,3-dimethyl-1,3,2-diazaphospholane-2-oxide; 2-chloromethyl-1,3-dimethyl-1,3,2-diazaphospholane-2-oxide; 2-trichloromethyl-1,3-dimethyl-1,3,2-diazaphospholane-2-oxide; 2-phenyl-1,3-dimethyl-1,3,2-diazaphospholane-2-oxide; 2-phenyl-1,3-dimethyl-1,3,2-diaza-phosphorinane-2-oxide; 2-benzyl-1,3-dimethyl-1,3,2-diazaphospholane-2-oxide; 2-allyl-1,3-dimethyl-1,3,2-diazaphospholane-2-oxide; 2-bromomethyl-1,3-dimethyl-1,3,2-diazaphospholane-2-oxide; 2-cyclohexyl-1,3-dimethyl-1,3,2-diazaphospholane-2-oxide; 2-(2-ethoxyethyl)-1,3-dimethyl-1,3,2-diazaphospholane-2-oxide; and 2-naphthyl-1,3-dimethyl-1,3,2-diazaphospholane-2-oxide.

(c) Triaryl arsines wherein the aryl groups are free from substituents containing reactive hydrogen atoms, said arsine being represented by the formula:

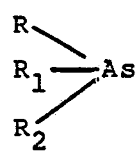

wherein each of R, $R_1$ and $R_2$ represents the same or different aryl moieties having from 6 to 12 carbon atoms, inclusive. Such compounds are described in U. S. Patent 3,406,198. Representative examples are: triphenylarsine, tris(p-tolyl)arsine, tris(p-methoxyphenyl)arsine, tris(p-ethoxyphenyl)arsine, tris(p-chlorophenyl)arsine, tris(p-fluorophenyl)arsine, tris(2,5-xylyl)arsine, tris(p-cyanophenyl)arsine, tris(1-naphthyl)arsine, tris(p-methylmercaptophenyl)arsine, tris(p-biphenylyl)arsine, p-chlorophenylbis-(p-tolyl)arsine and phenyl(p-chlorophenyl)(p-bromophenyl)-arsine.

(d) Also included are compounds of the formula:

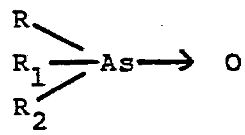

wherein each R, $R_1$ and $R_2$ represents the same or different alkyl or aryl groups having from 6 to 12 carbon atoms, inclusive. Representative examples of such are: triphenylarsine oxide, triethylarsine oxide, and polymer bound arsine oxide such as are described in U. S. Patent 4,143,063:

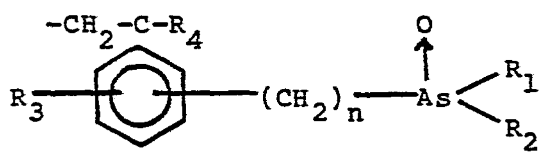

wherein $R_1$ and $R_2$ are hydrocarbyl from 1 to 12 carbon atoms inclusive, $R_3$ is hydrogen, chloro or methyl, $R_4$ is hydrogen or methyl, and n is 0 or 1.

(e) Metallic derivatives of acetylacetone such as the beryllium, aluminum, zirconium, chromium, and iron derivatives thereof as disclosed in U. S. Patent 3,152,131.

(f) Phosphate esters of the formula:

$$(RO)_3PO$$

wherein R is hydrocarbyl from 1 to 12 carbon atoms, inclusive. Such esters and methods for their preparation are disclosed in U. S. Patent 3,056,835. Representative examples are trimethylphosphate, triethylphosphate, ethyldipropylphosphate, triisopropylphosphate, triallylphosphate, triphenylphosphate, and tricresylphosphate.

(g) Phosphine oxides of the formula:

$$R_3PO$$

wherein R is hydrocarbyl from 1 to 12 carbon atoms, inclusive. Representative examples are triethylphosphine oxide, tributylphosphine oxide, triphenylphosphine oxide, and tris(chloromethyl)phosphine oxide.

(h) Metal complexes derived from a d-group transition element and $\pi$-bonding ligand selected from the group consisting of carbon monoxide, nitric oxide, hydrocarbylisocyanides, trihydrocarbylphosphine, trihydrocarbylarsine, trihydrocarbylstilbine, and dihydrocarbylsulfide wherein hydrocarbyl in each instance contains from 1 to 12 carbon atoms, inclusive, provided that at least one of the $\pi$-bonding ligands in the complex is carbon monoxide or hydrocarbylisocyanide. Such complexes and methods for the preparation are disclosed in U. S. Patent 3,406,197. Representative examples of such complexes are iron pentacarbonyl, di-iron pentacarbonyl, tungsten hexacarbonyl,

molybdenum hexacarbonyl, chromium hexacarbonyl, dimanganese decacarbonyl, nickel tetracarbonyl, ruthenium pentacarbonyl, and the complex of iron tetracarbonyl:methylisocyanide.

The term "hydrocarbyl" from 1 to 12 carbon atoms inclusive employed herein means the monovalent radical obtained by removing one hydrogen atom from a parent hydrocarbon having the stated carbon atom content. Illustrative of such groups are alkyl such as methyl-, ethyl-, propyl-, butyl-, pentyl-, hexyl-, heptyl-, octyl-, nonyl-, decyl-, undecyl-, undodecyl-, including isomeric forms thereof; alkenyl such as allyl, butenyl, pentenyl, hexenyl, heptenyl, octenyl, nonenyl, decenyl, undecenyl, and dodecenyl, including isomeric forms thereof; cycloalkyl such as cyclobutyl, cyclopentyl, cyclohexyl, cycloheptyl, cyclooctyl, and the like; cycloalkenyls such as cyclopentenyl, cyclohexenyl, cycloheptenyl, and the like; aralkyl such as benzyl, phenethyl, phenylpropyl, benzhydryl, naphthylmethyl, and the like; and aryls such as phenyl, tolyl, xylyl, naphthyl, biphenylyl, and the like.

The term "lower alkyl", as used herein, means alkyl from 1 to 6 carbon atoms, inclusive, such a methyl, ethyl, propyl, butyl, pentyl, hexyl and isomeric forms thereof.

The preferred carbodiimide catalysts are the 3-phospholene oxides. The most preferred carbodiimide

catalysts are the 1-aryl-3-lower alkyl-3-phospholene 1-oxide and 1,3-di(lower alkyl)-3-phospholene 1-oxide. The most preferred species are 1-phenyl-3-methyl-3-phospholene 1-oxide and 1-ethyl-3-methyl-3-phospholene-1-oxide, and the tris-(chloromethyl)phosphine oxide. Organotin compounds may also be employed in the present invention.

In accordance with the present invention, rigid, semi-flexible, flexible and microcellular foams may be prepared by the reaction of the organic diisocyanate compositions with polyols in the presence of urethane catalysts, blowing agents, surfactants and other additives which may be deemed necessary. Typical polyols which may be employed in the preparation of the foams of the instant invention include polyhydroxyl-containing polyesters, polyoxyalkylene polyether polyols, polyhydroxy-terminated polyurethane polymers, polyhydroxyl-containing phosphorus compounds, and alkylene oxide adducts of polyhydric sulfur-containing esters, polyacetals, aliphatic polyols or diols, ammonia, and amines including aromatic, aliphatic and heterocyclic amines as well as mixtures thereof. Alkylene oxide adducts of compounds which contain two or more different groups within the above-defined classes may also be used such as amino alcohols which contain an amino group and a hydroxyl group. Also, alkylene oxide adducts of compounds which contain one -SH group and one -OH group as

well as those which contain an amino group and a -SH group may be used. Generally, the equivalent weight of the polyols will vary from 53 to 10,000, preferably from 53 to 1000.

Any suitable hydroxy-terminated polyester may be used such as are obtained, for example, from polycarboxylic acids and polyhydric alcohols. Any suitable polycarboxylic acid may be used such as oxalic acid, malonic acid, succinic acid, glutaric acid, adipic acid, pimelic acid, suberic acid, azelaic acid, sebacic acid, brassylic acid, thapsic acid, maleic acid, fumaric acid, glutaconic acid, α-hydromuconic acid, β-hydromuconic acid, α-butyl-α-ethyl-glutaric acid, α, β-diethylsuccinic acid, isophthalic acid, terphthalic acid, phthalic acid, hemimellitic acid, and 1,4-cyclohexanedicarboxylic acid. Any suitable polyhydric alcohol may be used such as ethylene glycol, propylene glycol, trimethylene glycol, 1,2-butanediol, 1,3-butanediol, 1,4-butanediol, 1,2-pentanediol, 1,4-pentanediol, 1,5-pentanediol, 1,6-hexanediol, 1,7-heptanediol, glycerol, 1,1,1-trimethylol-propane, 1,1,1-trimethylolethane, 1,2,6-hexanetriol, α-methyl glucoside, pentaerythritol, and sorbitol. Also included within the term "polyhydric alcohol" are compounds derived from phenol such as 2,2-bis(4-hydroxyphenyl)-propane, commonly known as Bisphenol A.

Any suitable polyoxyalkylene polyether polyol may be used such as the polymerization product of an alkylene oxide with a polyhydric alcohol. Any suitable polyhydric alcohol may be used such as those disclosed above for use in the preparation of the hydroxy-terminated polyesters. Any suitable alkylene oxide may be used such as ethylene oxide, propylene oxide, butylene oxide, amylene oxide, and mixtures of these oxides. The polyalkylene polyether polyols may be prepared from other starting materials such as tetrahydrofuran and alkylene oxide-tetrahydrofuran mixtures; epihalohydrins such as epichlorohydrin; as well as aralkylene oxides such as styrene oxide. The polyalkylene polyether polyols may have either primary or secondary hydroxyl groups. Included among the polyether polyols are polyoxyethylene glycol, polyoxypropylene glycol, polyoxybutylene glycol, polytetramethylene glycol, block copolymers, for example, combinations of polyoxypropylene and polyoxyethylene glycols, poly-1,2-oxybutylene and polyoxyethylene glycols, poly-1,4-tetramethylene and polyoxyethylene glycols, and copolymer glycols prepared from blends or sequential addition of two or more alkylene oxides. The polyalkylene polyether polyols may be prepared by any known process such as, for example, the process disclosed by Wurtz in 1859 and Encyclopedia of Chemical Technology, Vol. 7, pp. 257-262, published by Interscience Publishers, Inc. (1951)

or in U. S. Patent No. 1,922,459. Polyethers which are preferred include the alkylene oxide addition products of trimethylolpropane, glycerine, pentaerythritol, sucrose, sorbitol, propylene glycol, and 2,2-bis(4-hydroxyphenyl)-propane and blends thereof having equivalent weights of from 100 to 5000.

Suitable polyhydric polythioethers which may be condensed with alkylene oxides include the condensation product of thiodiglycol or the reaction product of a dicarboxylic acid such as is disclosed above for the preparation of the hydroxyl-containing polyesters with any other suitable thioether glycol.

The hydroxyl-containing polyester may also be a polyester amide such as is obtained by including some amine or amino alcohol in the reactants for the preparation of the polyesters. Thus, polyester amides may be obtained by condensing an amino alcohol such as ethanolamine with the polycarboxylic acids set forth above or they may be made using the same components that make up the hydroxyl-containing polyester with only a portion of the components being a diamine such as ethylene diamine.

Polyhydroxyl-containing phosphorus compounds which may be used include those compounds disclosed in U. S. Patent No. 3,639,542. Preferred polyhydroxyl-containing phosphorus compounds are prepared from alkylene oxides and

acids of phosphorus having a $P_2O_5$ equivalency of from about 72 percent to about 95 percent.

Suitable polyacetals which may be condensed with alkylene oxides include the reaction product of formaldehyde or other suitable aldehyde with a dihydric alcohol or an alkylene oxide such as those disclosed above.

Suitable aliphatic thiols which may be condensed with alkylene oxides include alkanethiols containing at least two -SH groups such as 1,2-ethanedithiol, 1,2-propanedithiol, 1,2-propanedithiol, and 1,6-hexanedithiol; alkene thiols such as 2-butene-1,4-dithiol; and alkyne thils such as 3-hexyne-1,6-dithiol.

Suitable amines which may be condensed with alkylene oxides include aromatic amines such as aniline, o-chloro-aniline, p-aminoaniline, 1,5-diaminonaphthalene, methylene dianiline, the various condensation products of aniline and formaldehyde, and the isomeric diaminotoluenes; aliphatic amines such as methylamine, triisopropanolamine, ethylenediamine, 1,3-diaminopropane, 1,3-diaminobutane, and 1,4-diaminobutane.

The polyurethane foams of the present invention may also be prepared by the reaction of a graft copolymer polyol with the diisocyanate of the instant invention in the presence of a blowing agent and optionally in the presence of additional polyhydroxyl-containing components, chain-extending agents, catalysts, surface-active agents, stabilizers, dyes, fillers and pigments. Suitable processes for the preparation of cellular polyurethane products are disclosed in U. S. Reissue Patent 24,514 together with suitable machinery to be used in conjunction therewith. For the preparation of microcellular foams, blowing agents are generally not necessary. If desired for more expanded foams, they may be employed. When water is added as the blowing agent, corresponding quantities of excess isocyanate to react with the water and produce carbon dioxide may be used.

It is possible to proceed with the preparation of the polyurethane products by a prepolymer technique wherein an excess of organic diisocyanate of the instant invention is reacted in a first step with a polyol to prepare a prepolymer having free isocyanate groups which is then reacted in a second step with a polyol or an amine and a blowing agent such as water or a fluorocarbon to prepare a foam. Alternately, the components may be reacted in a single working step commonly known as the "one-shot" technique of preparing polyisocyanurate-polyurethanes products. Furthermore, instead of water, low boiling hydrocarbons such as pentane, hexane, heptane, pentene, and heptene; azo compounds such as azohexahydrobenzodinitrile; halogenated hydrocarbons such as dichlorodifluoromethane,

trichlorofluoromethane, dichlorodifluoroethane, vinylidene chloride, and methylene chloride may be used as blowing agents.

Chain-extending agents which may be employed in the preparation of the polyurethane foams include those compounds having at least two functional groups bearing active hydrogen atoms such as water, hydrazine, primary and secondary diamines, amino alcohols, amino acids, hydroxy acids, glycols, or mixtures thereof. A preferred group of chain-extending agents includes water, ethylene glycol, 1,4-butanediol, and primary and secondary diamines which react more readily with the polyisocyanates of the instant invention than does water. These include phenylenediamine, ethylenediamine, diethylenetriamine, N-(2-hydroxypropyl)-ethylenediamine, N,N'-di(2-hydroxypropyl)ethylenediamine, piperazine, and 2-methyl-piperazine.

Any suitable polyurethane promoting catalyst may be used including tertiary amines such as, for example, triethylenediamine, N-methylmorpholine, N-ethylmorpholine, diethylaminoethanol, N-laurylmorpholine, 1-methyl-4(di-methylaminoethyl) piperazine, 3-methoxy-N,N'-dimethylpropylamine, N,N,N'-trimethyl-isopropylproylenediamine, N,N,N',N"-tetraethylpropylenediamine, dimethylbenzylamine, dimethyl-cycloheylamiine and the like. Other suitable catalysts are, for example, tin compounds such as stannous chloride, tin

salts of carboxylic acids, such as dibutyltin di-2-ethyl hexanoate and stannous octoate, as well as other organo metallic compounds such as are disclosed in U. S. Patent no. 2,846,408. The preferred catalysts for the preparation of polyurethane foams are triethylenediamine, N-methylmorpholine and N-ethylmorpholine and dibutyltin dilaurate.

If desired, a surface-active agent may be employed. Numerous surface-active agents have been found satisfactory. Nonionic surface-active agents are preferred. Of these, the nonionic surface-active agents prepared by the sequential addition of propylene oxide and then ethylene oxide to propylene glycol and the solid or liquid organosilicones have been found particularly desirable. Other surface-active agents which are operative, although not preferred, include polyethylene glycol ethers of long chain alcohols, tertiary amine or alkylolamine salts of long chain alkyl acid sulfate esters, alkylsulfonic esters, and alkylarylsulfonic acids.

The following examples illustrate the nature of the invention. All parts are by weight unless otherwise stated.

Example 1

Pure 4,4'-diphenylmethane diisocyanate, 100 pbw was melted, charged into a reactor and maintained at 40° to 50°C. Carbodiimide-uretonimine modified 4,4'-diphenylmethane diisocyanate 25 pbw was added to the molten product and stored at 25° to 30°C. The composition of the mixture was 93.5 percent pure 4,4'-diphenylmethane diisocyanate and 6.5 percent carbodiimide-uretonimine modified 4,4'-diphenylmethane diisocyanate. This product was stored for nine months at 25°C with a dimer formation of only about 0.006 percent per week. Pure 4,4'-diphenylmethane diisocyanate stored at 45°C formed dimers at 0.035 percent per week. This indicates the extended shelf storage capability of modified 4,4'-diphenylmethane diisocyanate.

The embodiments of the invention in which an exclusive privilege or property is claimed are defined as follows:

1. A 4,4'-diphenylmethane diisocyanate composition which is storage stable at temperatures of about 25°C which composition contains from about 2 to about 10 weight percent uretonimine structure.

2. The diisocyanate composition of claim 1 wherein the composition contains from about 5 to about 8 weight percent uretonimine structure.

3. A rigid polyurethane foam prepared by the reaction of the polyisocyanate composition of claim 1 with a polyol in the presence of a catalyst, a blowing agent, and other additives.

4. A rigid polyurethane foam prepared by the reaction of the polyisocyanate composition of claim 2 with a polyol in the presence of a catalyst, a blowing agent, and other additives.

5. A semi-flexible polyurethane foam prepared by the reaction of the polyisocyanate composition of claim 1 with a polyol in the presence of a catalyst, a blowing agent, and other additives.

6. A semi-flexible polyurethane foam prepared by the reaction of the polyisocyanate composition of claim 2 with a polyol in the presence of a catalyst, a blowing agent, and other additives.